# FASCICULE DE BREVET EUROPEEN

(11) **EP 1 563 868 B1**
(45) Date de publication et mention de la délivrance du brevet: **24.11.2010**
(21) Numéro de dépôt: 05300119.4
(22) Date de dépôt: 15.02.2005
(51) Int. Cl.: A61N 1/18, A61N 1/30

(54) **Kit de traitement comportant une structure composite et une tête excitatrice**
Behandlungskit mit Verbundstruktur und Erregerkopf
Treatment kit comprising a composite structure and an excitation head

(30) Priorité: 16.02.2004 FR 0450279
(43) Date de publication de la demande: 17.08.2005
(73) Titulaire: L'Oréal, 75008 Paris (FR)
(72) Inventeur: Guéret, Jean-Louis, 75016, PARIS (FR)
(74) Mandataire: Tanty, François

(56) Documents cités:
- EP-A- 0 617 979
- WO-A-01/03638
- FR-A- 2 256 750
- FR-A- 2 722 696
- US-A- 5 087 240
- US-A1- 2002 147 467
- US-A1- 2004 006 374
- US-B1- 6 629 968

## Description

La présente invention concerne le traitement du corps ou du visage au moyen d'une structure composite renfermant un actif notamment cosmétique ou dermatologique.

On connaît par la demande internationale WO 02/24274 un dispositif comportant une poche pourvue d'électrodes qui peuvent être connectées à un générateur et d'une ouverture permettant l'introduction dans la poche d'un liquide à administrer.

La demande US 2002/0198484 a pour objet un dispositif de distribution transdermique par transport électrique d'une substance comportant dans un réceptacle deux électrodes et deux réservoirs. Les électrodes sont dès l'origine en contact avec les réservoirs.

Le document US 472977 décrit un vêtement qui délivre ou reçoit des impulsions électriques grâce à des électrodes fixées dans ledit vêtement. La différence avec la présente invention réside en ce que les électrodes sont solidaires à la structure composite.

L'invention est comme définie dans le jeu de revendications.

L'invention offre de nombreux avantages.

Tout d'abord, elle permet de disposer d'un kit de traitement permettant le déplacement aisé et sans contrainte des électrodes par rapport à la structure composite. Cela permet, par exemple, d'adapter le traitement à la sensibilité de la zone à traiter. L'invention peut aussi permettre de favoriser l'action et/ou la pénétration de l'actif dans la zone traitée. La circulation du courant entre les électrodes peut par exemple favoriser l'ouverture et la dilatation des pores de la peau et faciliter l'action et/ou la pénétration dans la peau de l'actif

L'invention peut également permettre, le cas échéant, d'activer la microcirculation sanguine, d'améliorer le tonus musculaire ou la capacité de cicatrisation de la peau.

En outre, l'invention peut permettre, en réalisant la structure composite de telle sorte que les électrodes soient sans contact substantiel avec l'actif lorsqu'elles sont au contact de la face extérieure de la structure composite, de ne pas les salir outre mesure, ce qui peut permettre de réduire le risque de formation d'un dépôt susceptible, entre autres, de nuire à la conduction de l'électricité.

L'invention peut encore offrir la possibilité à l'utilisateur de faire varier l'intensité du courant électrique en appliquant plus ou moins les électrodes sur la structure composite.

La structure composite peut être par exemple l'un d'un patch, d'un masque ou d'une serviette.

La structure composite peut comporter une couche extérieure hydrophile, apte à être mise en contact avec les électrodes. Cette couche extérieure peut être humidifiée en début de traitement et permettre ainsi la circulation du courant. A l'état sec, la couche extérieure peut être non conductrice de l'électricité.

La structure composite peut comporter au moins une couche formée d'un tissé. La structure composite peut encore comporter au moins une couche formée d'un non-tissé, notamment deux couches extrêmes formées chacune d'un non-tissé. La structure composite peut également comporter au moins une couche d'une matrice gélifiée et/ou polymérique, laquelle peut être une couche réservoir contenant l'actif et se situer entre les deux couches extrêmes, par exemple.

Il existe diverses possibilités tendant à éviter que l'actif ne migre vers la face où sont appliquées les électrodes, afin de réduire le risque de salissure de ces dernières.

La structure composite peut comporter par exemple une couche réservoir contenant l'actif, prise en sandwich entre deux couches extrêmes. Au moins celle destinée à venir au contact des électrodes peut être dépourvue de l'actif

Les deux couches extrêmes peuvent avoir par exemple des épaisseurs et/ou des porosités et/ou d'autres propriétés, notamment d'affinité pour l'eau ou l'actif contenu dans la couche réservoir, qui diffèrent.

Les électrodes peuvent par exemple être amenées au contact de la couche extrême d'épaisseur la plus importante. En variante ou additionnellement, les électrodes peuvent être amenées au contact de la couche ayant la porosité la plus faible.

La porosité de la couche défmissant la face extérieure de la structure composite peut par exemple être choisie de telle sorte que l'actif diffuse difficilement dans cette couche. Ainsi, les électrodes peuvent demeurer, au cours de l'utilisation de la structure composite, substantiellement sans contact avec l'actif.

La structure composite peut être agencée pour permettre d'identifier visuellement la face destinée à venir en contact avec la peau ou au contraire celle destinée à venir au contact des électrodes. La structure composite peut notamment présenter des faces ayant des couleurs différentes.

La structure composite peut être sensiblement sèche avant l'utilisation et être mouillée pour être rendue conductrice.

Avant l'utilisation, l'actif peut être à l'état déshydraté, lyophilisé et/ou cristallisé. L'actif peut présenter une forme particulaire ou non.

La structure composite peut encore comporter une couche imprégnée d'un liquide, notamment d'eau, d'alcool ou de propylène glycol. La couche imprégnée de liquide peut être la couche réservoir comportant l'actif.

La structure composite peut être non-adhésive à sec et adhésive lorsqu'elle est humidifiée.

La structure composite peut contenir tous éléments conducteurs d'électricité, par exemple un électrolyte.

La structure composite peut comporter des particules magnétiques et/ou des particules électriquement conductrices. Les particules magnétiques peuvent être, le cas échéant, enrobées. Elles peuvent comporter des fibres.

En variante, la structure composite peut être dépourvue de particules magnétiques et/ou électriquement conductrices, la conduction s'effectuant par exemple grâce à la présence d'ions.

Les particules électriquement conductrices et/ou magnétiques peuvent être localisées dans la couche réservoir ou dans une autre couche de la structure. Les particules peuvent par exemple être prises entre les fibres d'un tissé, d'un non-tissé, ou dispersées dans une matrice de la structure composite, notamment une matrice gélifiée et/ou polymérique.

La structure composite peut comporter une couche réservoir contenant l'actif, prise en sandwich entre une couche hydrophile et une couche hydrophile ou hyrophobe.

La couche réservoir peut comporter un adhésif.

L'une au moins des couches extérieures à la couche réservoir peut être soudée sur celle-ci.

La couche réservoir peut comporter l'un au moins d'un composé superabsorbant, d'un polyacrylate, d'un polymère acrylique, vinylique, d'un polyuréthane, d'un latex, ou pseudolatex.

L'actif peut être un actif cosmétique ou dermatologique ou être autre et destiné à être administré à l'être humain.

L'actif peut comporter l'un au moins d'un composé choisi dans la liste suivante : métaux et leurs alliages, cobalt, baryum, chrome, aluminium, argent, cuivre, titane, bronze, manganèse, oxydes métalliques, oxydes de fer, notamment ferrite, terres rares, silicates, sulfates, notamment sulfate de baryum, carbonates, notamment carbonate de calcium, composés non ferreux, notamment soufre, magnésium, calcium, bore, potassium, carbone, oligo-éléments, sel marin, sels gemmes, argile, stéatite, algues et planctons et leurs extraits, racines, réglisse, gingembre, cires huileuses, protéines, hormones, collagènes, aluns, notamment pierre d'alun, glucose, vitamines, notamment vitamine C, vitamine A, vitamine F, vitamine B, vitamine E, extraits végétaux, glycérine, laponite, tensioactifs, collagène, acides, notamment acide salicylique, acide tio, caféine, huiles essentielles aromatiques, colorants, anti-oxydants, anti-radicaux libres, hydroabsorbants, hydratants, dépigmentants, liporégulateurs, anti-acnéique, anti-séborrhéiques, agents anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, nourrissants.

Les électrodes peuvent être montées avec une possibilité de déplacement par rapport au reste de l'excitateur, notamment de coulissement ou de rotation.

L'une au moins des électrodes, mieux les deux électrodes, peuvent être orientables par rapport à l'organe de préhension.

L'écartement entre au moins deux électrodes peut être réglable. Au moins une électrode peut être amovible.

Le kit peut par exemple comporter une pluralité d'électrodes amovibles de formes différentes, par exemple des électrodes dont la surface de contact avec la structure composite est différente, de manière à pouvoir être sélectionnées et fixées sur l'excitateur en fonction du traitement à effectuer, de la structure composite choisie et/ou de la zone de la peau traitée.

En variante, l'excitateur électrique peut être dépourvu d'électrodes amovibles.

Les électrodes peuvent être maintenues immobiles l'une par rapport à l'autre par un isolant électrique, notamment un matériau tel qu'une résine thermoplastique ou élastomère.

Les électrodes peuvent être dépourvues d'arête vive. Chaque électrode peut par exemple comporter une extrémité arrondie, notamment sphérique.

L'excitateur électrique peut être alimenté par l'un au moins d'une pile électrique ou d'un accumulateur. En variante, l'excitateur électrique peut comporter des moyens de raccordement à un adaptateur électrique branché sur le secteur.

L'excitateur électrique peut présenter un boîtier ayant une forme ergonomique, par exemple facilement manipulable d'une seule main. En variante, l'organe servant à la préhension de l'excitateur électrique est constitué au moins en partie par une pile ou un accumulateur.

L'excitateur électrique peut comporter un interrupteur, ou au contraire en être dépourvu.

L'excitateur électrique peut comporter un témoin lumineux qui s'allume lorsque le courant circule dans les électrodes. Ce témoin lumineux peut comporter par exemple une diode électroluminescente bicolore.

La circulation du courant peut par exemple être intermittente.

Lors de l'utilisation, la tension aux bornes des électrodes peut être identique à la tension aux bornes d'une pile ou d'un accumulateur logé dans l'excitateur électrique. En variante, l'excitateur électrique peut comporter des moyens pour élever ou réduire la tension délivrée par la source d'énergie électrique.

La tension aux bornes des électrodes est de préférence inférieure à 30 V, par exemple de l'ordre de 9 V.

L'excitateur électrique peut comporter un organe de réglage de la puissance, par exemple de la tension et/ou du courant maximal, notamment un potentiomètre ou un contacteur à plusieurs positions.

L'excitateur électrique peut comporter des moyens pour mettre en vibration les électrodes, permettant de transmettre des vibrations à la structure composite et à la région du corps sur laquelle celle-ci est appliquée.

L'excitateur électrique peut comporter un sélecteur permettant à l'utilisateur de choisir s'il souhaite ou non faire vibrer les électrodes.

Le cas échéant, le sélecteur peut être agencé pour permettre aux électrodes de vibrer sans que celles-ci ne soient alimentées électriquement.

Les moyens pour mettre en vibration les électrodes peuvent comporter un moteur électrique entraînant en rotation une masse non équilibrée.

Le cas échéant, l'excitateur électrique peut comporter des moyens, tels que par exemple un potentiomètre, permettant de régler l'amplitude et/ou la fréquence de vibration des électrodes.

L'excitateur électrique est avantageusement étanche.

La fourniture de la structure composite et/ou de l'excitateur électrique, seuls ou en combinaison, par exemple rassemblés dans un coffret, peut s'effectuer par tout canal de vente, notamment par la vente dans un magasin ou par correspondance, ou par le biais d'un institut de beauté ou d'un salon de coiffure, par exemple.

L'excitateur électrique et la structure composite peuvent être séparés l'un de l'autre, et le kit de traitement peut être configuré de telle sorte que lesdites au moins deux électrodes peuvent être amenées sélectivement en contact avec la structure composite.

Les deux électrodes peuvent être solidaires d'un organe de préhension et peuvent être amenées simultanément, par le maniement de l'organe de préhension, au contact de la deuxième face de la structure composite.

Les deux électrodes peuvent faire passer un courant électrique dans la structure composite et/ou lui transmettre des vibrations.

Les électrodes peuvent être interchangeables, de manière par exemple à permettre à l'utilisateur de choisir entre plusieurs électrodes selon le type de traitement souhaité. Par exemple, certains éléments peuvent être électriquement conducteurs et permettre la formation d'électrodes et d'autres non.

La methode de l'invention est définie dans la revendication 47.

L'invention se rapporte à une méthode cosmétique, non-thérapeutique. De ce fait, l'actif ne peut dans ce cas pas comporter un composé de la liste suivante: hormones, acides notamment acide salicylique, anti-radicaux libres, tensioactifs, anti-acnéique, antiséborrhéiques, antiinflammatoires, cicatrisants, protecteurs vasculaires, anti-bactériens, antifongiques, insensibilisants, ou immunomodulateurs.

Par « sans contact substantiel », il faut comprendre que lorsque l'on retire les électrodes de la structure composite, il n'y a pas de résidu important d'actif sur celles-ci.

Le procédé peut comporter en outre l'humidification de la structure composite avant son application sur la zone de la peau à traiter et/ou l'humidification de la peau préalablement à l'application de la structure composite.

On peut par exemple appliquer la structure composite sèche sur une zone de la peau préalablement humidifiée.

On peut maintenir les électrodes fixes par rapport à la structure composite durant le traitement. En variante, on peut déplacer les électrodes par rapport à la structure composite durant le traitement.

On peut appliquer les électrodes sur la structure composite de façon permanente durant tout le traitement. En variante, on peut appliquer les électrodes sur la structure composite de façon intermittente durant le traitement.

On peut par exemple appliquer au total les électrodes sur la structure composite pendant une durée comprise entre 3 et 15 mn.

Dans un exemple de mise en oeuvre de l'invention, l'on met en vibration les électrodes durant le traitement.

La structure composite peut être à usage unique et jetée après utilisation.

Indépendamment ou en combinaison avec ce qui précède, l'invention a encore pour objet un procédé pour promouvoir la vente d'au moins une structure composite à appliquer sur la peau, en faisant état de la possibilité d'utiliser la structure composite en association avec un courant électrique.

Une telle promotion pourra se faire par n'importe quel canal de communication. Elle pourra être faite notamment par un vendeur, directement sur un point de vente, par la radio, la télévision ou le téléphone, notamment dans le cadre de spots publicitaires ou de messages courts. Elle pourra être faite également par le canal de la presse écrite ou par le biais de tout autre document, en particulier à des fins publicitaires. Elle pourra se faire également par Internet, par tout autre réseau informatique adéquat ou par un réseau de téléphonie mobile. Elle pourra être faite également directement sur le produit, notamment sur son packaging ou sur toute notice explicative qui lui est associée.

L'invention pourra être mieux comprise à la lecture de la description détaillée qui va suivre, d'exemples non limitatifs de celle-ci, et à l'examen du dessin annexé, sur lequel :
- la figure 1 représente de manière schématique un excitateur électrique dont les électrodes sont au contact d'une structure composite appliquée sur la peau,
- la figure 2 est une vue de dessus de l'excitateur électrique de la figure 1,
- la figure 3 est une vue analogue à la figure 2 avec une variante de réalisation des électrodes,
- les figures 4 et 5 illustrent l'utilisation de structures composites avec l'excitateur électrique,
- les figures 6 et 7 représentent en coupe, de manière schématique, des variantes de réalisation de la structure composite,
- les figures 8 à 10 représentent des exemples de sources d'énergie électrique,
- les figures 11 à 17 représentent de manière schématique d'autres exemples d'excitateurs électriques,
- la figure 18 représente un coffret contenant un kit conforme à l'invention,
- la figure 19 représente de manière schématique une structure composite en forme de masque pour les yeux,
- la figure 20 est une vue, en perspective, d'une variante de réalisation,
- la figure 21 est une vue éclatée, en perspective, de l'excitateur électrique de la figure 20,
- les figures 22 et 23 sont respectivement des vues selon les flèches XXII et XXIII de l'excitateur électrique des figures 20 et 21, refermé,
- les figures 24 et 25 sont des coupes longitudinales respectivement selon XXIV-XXIV et XXV-XXV de l'excitateur électrique de la figure 20,
- la figure 26 représente une variante réalisée conformément à l'invention, et
- les figures 27 et 28 représentent une autre variante de réalisation, à l'arrêt dans le cas de la figure 27 et en fonctionnement dans le cas de la figure 28.

Sur les figures, les proportions relatives n'ont pas toujours été respectées, dans un souci de clarté du dessin.

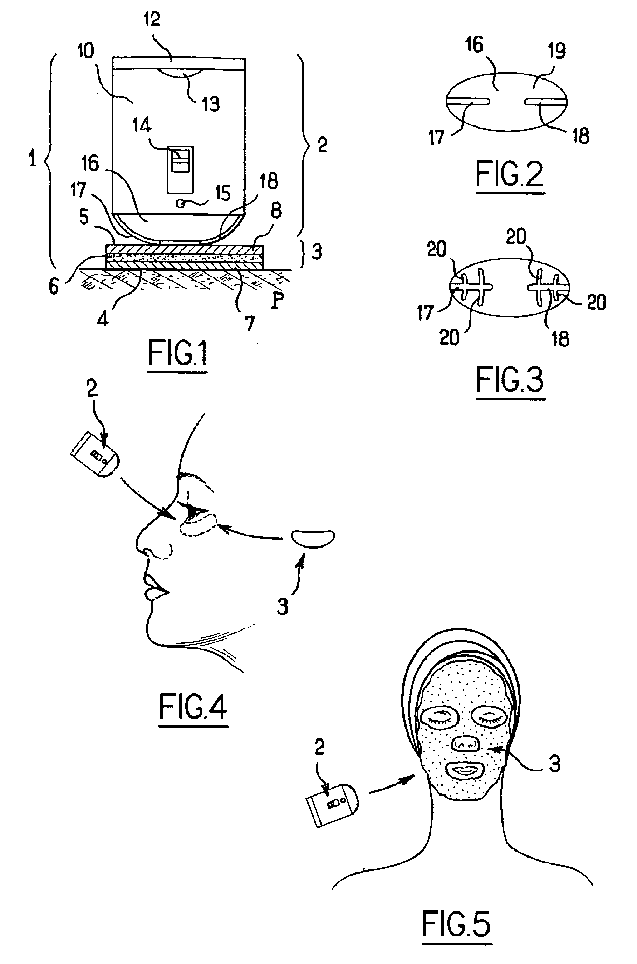

On a représenté à la figure 1 un ensemble 1 comportant un excitateur électrique 2 et une structure composite 3 destinée à être appliquée par une première face 4 sur une zone de peau P à traiter. La structure composite 3 comporte une deuxième face 5, opposée à la première, avec laquelle l'excitateur électrique 2 peut venir en contact.

### Structure composite

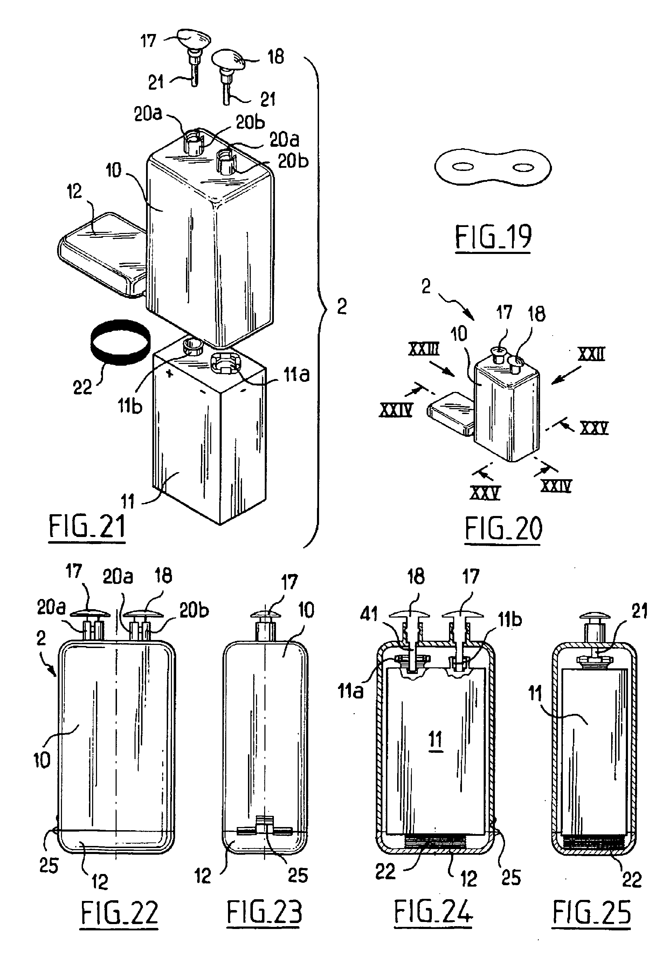

La structure composite 3 se présente par exemple sous la forme d'un patch, comme on le voit à la figure 4, d'un masque pour le visage comme illustré à la figure 5, ou encore d'un masque pour les yeux seulement, comme illustré à la figure 19, mais peut présenter d'autres formes encore, ayant par exemple un contour adapté à la forme de la zone à traiter, notamment une forme de disque, de bande ou de serviette, cette liste n'étant pas limitative.

La structure composite 3 comporte au moins un actif destiné à exercer une action sur la peau ou une action plus générale, cet actif pouvant, comme illustré sur la figure 1, être contenu dans une couche réservoir 6 située entre une première couche 7 et une deuxième couche 8 défmissant respectivement les première et deuxième faces 4 et 5.

La couche réservoir 6 peut notamment comporter une matrice adhésive assurant la cohésion des première et deuxième couches 7 et 8. Les propriétés de ces dernières sont choisies, dans l'exemple illustré, de manière à permettre, lorsque la structure composite 3 est utilisée, une migration préférentielle de l'actif vers la première face 4 plutôt que vers la deuxième face 5.

Pour parvenir à ce transfert sélectif de l'actif vers la première face 4, la première couche 7 peut par exemple être réalisée avec des pores larges tandis que la deuxième couche 8 est choisie moins poreuse que la première couche 7.

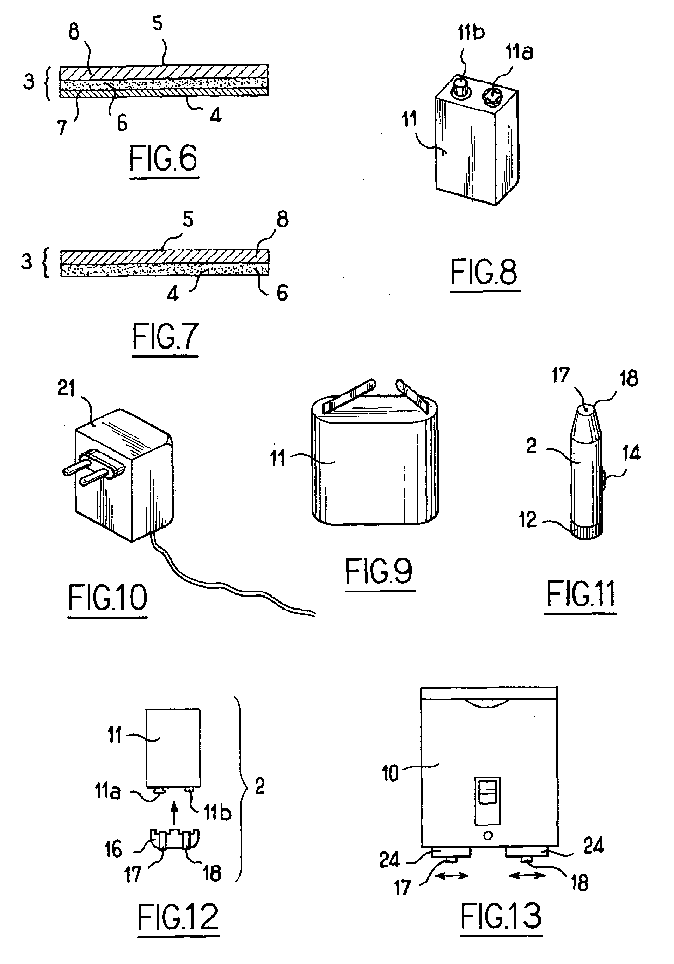

La structure composite 3 peut, comme illustré à la figure 6, présenter des première et deuxième couches 7 et 8 d'épaisseurs inégales, la deuxième couche 8 étant par exemple d'épaisseur supérieure à celle de la première couche 7, afin que le transfert de l'actif ait lieu préférentiellement vers la première face 4 plutôt que vers la deuxième face 5. La deuxième couche 8 peut par exemple être au moins deux fois plus épaisse que la première couche 7.

La première couche 7 peut encore ne pas exister, comme illustré à la figure 7, la couche réservoir 6 contenant l'actif définissant la première face 4. Dans cet exemple, la deuxième couche 8 recouvre la couche réservoir 6 et définit la deuxième face 5.

Les couches extrêmes 7 et 8 et la couche réservoir 6 peuvent être de diverses natures.

On peut utiliser par exemple pour l'une au moins de ces couches un tissé, un non-tissé, une matrice gélifiée ou polymérique ou d'autres supports encore, de préférence souples. Le cas échéant, l'une au moins des couches peut être au moins partiellement métallisée, par exemple aluminisée. On peut notamment utiliser un non-tissé aluminisé.

La couche réservoir 6 comporte par exemple un adhésif choisi dans la liste suivante : adhésif à base vinylique, à base de PVA ou de PVP, à base de pseudo latex, à base de polymères acryliques, de polyuréthanes ou d'élastomères de latex. La couche réservoir 6 peut comporter des absorbeurs d'humidité, par exemple des polyacrylates ou autres superabsorbants, capables d'absorber plusieurs fois leur poids de liquide.

La couche réservoir peut comporter par exemple au moins un polymère acrylique, vinylique, un polyuréthane, un latex ou pseudolatex.

La structure composite est par exemple réalisée conformément à la demande de brevet US 2001/0028894.

La structure composite 3 peut comporter des particules électriquement conductrices, par exemple d'un métal tel que du cuivre, qui permettent d'améliorer la conduction du courant électrique circulant entre les électrodes 17 et 18. Cela peut améliorer la qualité du traitement.

Ces particules métalliques peuvent par exemple être dispersées dans la couche réservoir 6 ou ailleurs, étant par exemple retenues entre les fibres d'un tissé ou d'un non-tissé.

Le cas échéant, la structure composite 3 peut comporter des particules magnétiques, éventuellement enrobées, afin de créer un champ magnétique.

Lors de l'utilisation, les effets apportés par les particules magnétiques peuvent se cumuler avec ceux liés au passage du courant électrique.

L'actif peut par exemple comporter ou être constitué par au moins un composé choisi dans la liste suivante : métaux et leurs alliages, cobalt, baryum, chrome, aluminium, argent, cuivre, titane, bronze, manganèse, oxydes métalliques, oxydes de fer, notamment ferrite, terres rares, silicates, sulfates, notamment sulfate de baryum, carbonates, notamment carbonate de calcium, composés non ferreux, notamment soufre, magnésium, calcium, bore, potassium, carbone, oligo-éléments, sel marin, sels gemmes, argile, stéatite, algues et planctons et leurs extraits, extraits végétaux, racines, réglisse, gingembre, cires huileuses, protéines, hormones, collagènes, aluns, notamment pierre d'alun, glucose, vitamines, notamment vitamine C, vitamine A, vitamine F, vitamine E, vitamine B, glycérine, laponite, tensioactifs, collagène, acides, notamment acide salicylique, acide tio, caféine, huiles essentielles aromatiques, colorants, anti-oxydants, anti-radicaux libres, hydroabsorbants, hydratants, dépigmentants, liporégulateurs, anti-acnéique, anti-séborrhéiques, agents anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, nourrissants.

La structure composite 3 se présente avantageusement à l'utilisateur sous une forme sensiblement sèche, ce qui peut faciliter son stockage.

Lorsque la structure composite 3 est sous une telle forme, les première et deuxième couches 7 et 8 peuvent ne pas présenter une conduction électrique significative, la structure composite 3 étant destinée à être mouillée lors de l'utilisation.

### Excitateur électrique

L'excitateur électrique 2 comporte, dans l'exemple illustré, un boîtier 10 formant organe de préhension, qui loge une source électrique, par exemple une pile électrique 11, notamment une pile sèche de 9 V, représentée de manière schématique à la figure 8 ou une pile de 4,5 V représentée à la figure 9.

Bien entendu, l'invention n'est pas limitée à une source électrique particulière et l'on peut utiliser d'autres types de piles ou accumulateurs électriques, ou encore un adaptateur secteur 21 comportant un transformateur, représenté de manière schématique à la figure 10.

La tension délivrée par les électrodes est par exemple une tension continue, mais l'excitateur électrique pourrait comporter un circuit électronique permettant par exemple de délivrer un courant impulsionnel ou alternatif.

En prenant l'exemple de la figure 1, on voit que le boîtier 10 peut comporter un capot de fermeture 12 qui permet de remplacer la source électrique, un onglet 13 pouvant être réalisé sur le boîtier 10 pour faciliter l'enlèvement du capot 12. Le boîtier 10 peut aussi comporter un interrupteur électrique 14 et un témoin lumineux 15, comme on le voit sur la figure 1.

L'excitateur électrique 2 comporte une tête 16 comportant deux électrodes 17 et 18 formant saillie à sa surface, ces électrodes 17 et 18 étant par exemple maintenues solidaires l'une de l'autre par une masse 19 d'un matériau isolant, tel que par exemple une matière plastique relativement souple, notamment un élastomère silicone.

Les électrodes 17 et 18 sont reliées électriquement aux bornes de la source électrique contenue dans le boîtier 10 par des connexions non représentées.

Le témoin lumineux 15 peut s'allumer par exemple lorsqu'une tension est appliquée aux électrodes 17 et 18. En variante, le témoin lumineux 15 ne peut s'allumer que lorsqu'un courant électrique circule entre les électrodes 17 et 18. En variante encore, le témoin lumineux 15 peut s'allumer par exemple d'une première couleur lorsque les électrodes 17 et 18 sont sous tension et s'allumer d'une deuxième couleur lorsqu'un courant électrique circule entre les deux électrodes 17 et 18. Le témoin lumineux 15 peut alors être dans ce cas une diode électroluminescente bicolore.

On peut réaliser l'excitateur électrique 2 différemment, à commencer par exemple par les électrodes 17 et 18 qui peuvent présenter diverses formes et notamment des ramifications 20 comme illustré à la figure 3. Ces ramifications 20 peuvent par exemple permettre d'accroître la surface en contact électrique avec la structure composite 3 lors de l'utilisation.

De préférence, les électrodes 17 et 18 ne présentent pas d'arête vive, ce qui leur permet d'être déplacées sans accrocher la structure composite 3 et de ne pas griffer la peau.

L'excitateur électrique 2 peut être réalisé encore autrement, par exemple de la manière illustrée sur les figures 20 à 25.

Dans cet exemple de réalisation, l'excitateur électrique 2 comporte un boîtier 10 logeant une pile électrique 11 de 9V, étant dépourvu d'interrupteur électrique.

Le boîtier 10 comporte en outre un capot de fermeture 12 relié au reste du boîtier 10 par une charnière-film 25.

Les électrodes 17 et 18 comportent une tête arrondie à l'extrémité d'une tige 41, et sont réalisées dans un matériau élastomère conducteur. Elles sont maintenues chacune solidaires du boîtier 10 par deux parois 20a, 20b définissant un logement dans lequel peuvent être encliquetées les électrodes 17 et 18. Les tiges 41 des électrodes 17 et 18 traversent la paroi de dessus du boîtier 10 et viennent en appui contre les bornes électriques 11a et 11b de la pile 11.

Un ressort ou un bloc de mousse 22 disposé au fond du boîtier 10 permet de maintenir la pile 11 en contact électrique avec les tiges 41 des électrodes 17 et 18.

L'excitateur électrique 2 peut être réalisé autrement encore, par exemple sous la forme d'un stylo, comme illustré à la figure 11, avec deux électrodes 17 et 18 concentriques.

L'excitateur électrique 2 peut encore être formé par le simple assemblage de la pile 11 et de la tête 16, comme illustré à la figure 12. Dans ce cas, la pile 11 sert d'organe de préhension pour la manipulation de l'excitateur électrique 2.

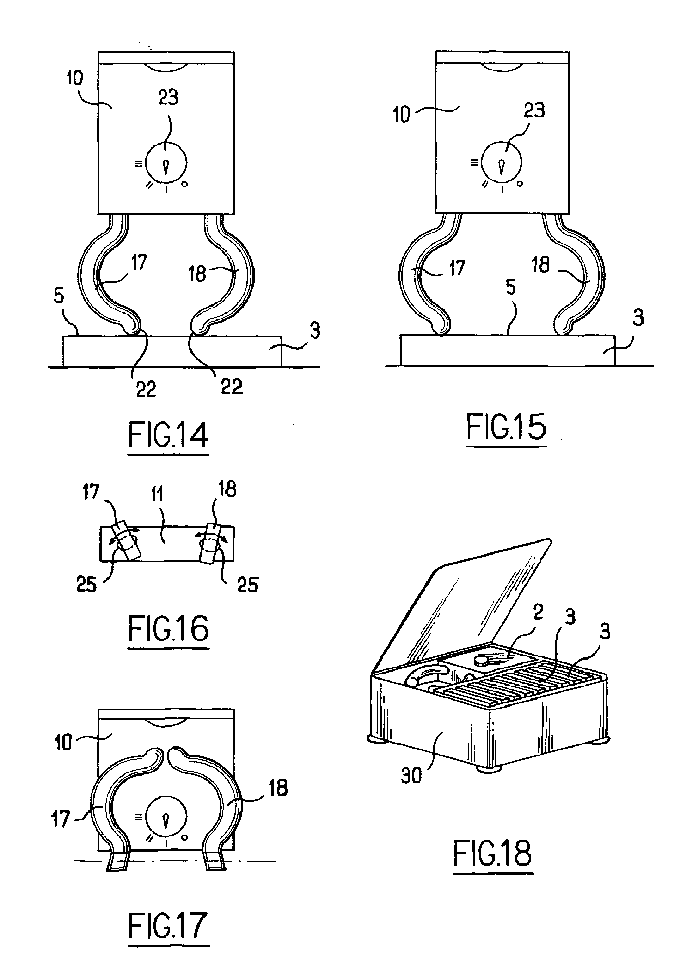

Les électrodes 17 et 18 peuvent être fixes relativement à la tête 16 ou, en variante, les électrodes 17 et 18 peuvent être montées sur l'excitateur électrique 2 avec une possibilité de réglage, par exemple en écartement, comme illustré aux figures 13 à 15 et/ou en orientation, comme illustré à la figure 16.

Dans l'exemple des figures 14 et 15, les électrodes 17 et 18 présentent des extrémités 22 arrondies et une forme générale arquée.

On a également illustré sur les figures 14 et 15 la possibilité pour l'excitateur électrique 2 d'avoir un organe de réglage 23 permettant par exemple de régler la tension aux bornes des électrodes 17 ou 18 et/ou le courant maximum qui peut circuler de l'une à l'autre lors de l'utilisation.

Dans l'exemple des figures 14 et 15, les électrodes 17 et 18 pivotent autour d'axes parallèles.

Dans la variante illustrée à la figure 13, les électrodes 17 et 18 peuvent coulisser, étant par exemple engagées dans des glissières 24.

Le cas échéant, les électrodes 17 et 18 peuvent être montées sur le boîtier 10 de manière à pouvoir se rabattre, en l'absence d'utilisation, dans une position facilitant le stockage et le transport de l'excitateur électrique 2, comme illustré à la figure 17.

Dans la variante de réalisation de la figure 16, les électrodes 17 et 18 sont indépendantes l'une de l'autre, étant fixées respectivement sur les bornes électriques 11a et 11b de la pile 11. Dans ce cas, c'est la pile 11 qui rend solidaires les électrodes 17 et 18 l'une de l'autre afin de permettre leur application simultanée sur la structure composite 3. Les électrodes 17 et 18 peuvent être reliées électriquement à des plots 25 qui sont agencés pour se fixer respectivement sur les bornes 11a et 11b de la pile 11.

Le cas échéant, comme illustré à la figure 18, l'excitateur électrique 2 peut être commercialisé avec une pluralité de structures composites 3 dans un emballage 30 qui se présente par exemple sous la forme d'un coffret. Chaque structure composite 3 peut être conditionnée individuellement ou non.

L'excitateur électrique 2 peut encore être vendu séparément, indépendamment des structures composites.

### Utilisation

Pour utiliser le kit 1, l'utilisateur peut, comme illustré à la figure 4, mouiller la structure composite 3, par exemple en la plaçant sous un robinet d'eau courante, chaude ou froide, puis l'appliquer sur la peau sur la zone à traiter. En variante, on peut tremper la structure composite 3 dans une solution autre que de l'eau, par exemple une solution salée ou hydroalcoolique ou hydrolipoalcoolique.

L'adhérence de la structure composite 3 sur la peau peut être due par exemple uniquement au fait que la structure composite 3 est mouillée, la structure composite 3 ne présentant à l'état sec aucune adhérence.

Les première et deuxième couches 7 et 8 permettent, lorsqu'imbibées d'eau, à la couche réservoir 6 de libérer son ou ses actifs. Ce ou ces actifs migrent préférentiellement vers la première face 4 plutôt que vers la deuxième face 5, comme expliqué ci-dessus.

A cet instant, les électrodes 17 et 18 ne sont pas au contact de la structure composite 3.

Une fois la structure composite 3 appliquée sur la peau, l'utilisateur peut amener les électrodes 17 et 18 au contact de la deuxième face 5 et provoquer le passage d'un courant électrique dans la structure composite 3, ainsi que dans la peau. Le passage de ce courant peut favoriser la micro-circulation sanguine notamment et avoir éventuellement un effet sur la pénétration du ou des actifs dans la peau. L'utilisateur peut, le cas échéant, ressentir des picotements lors de l'application des électrodes 17 et 18 sur la structure composite 3, ces picotements étant dus au passage du courant dans la peau.

Les électrodes 17 et 18 peuvent être amenées d'un geste simple au contact de la structure composite 3, par manipulation de l'organe de préhension 10.

On peut par exemple traiter en continu une zone très précise de la peau.

L'utilisateur peut encore déplacer les électrodes 17 et 18 sur la structure composite 3 au cours de l'utilisation, de façon à faire varier par exemple la localisation des lignes de courant électrique.

Lorsque les électrodes 17 et 18 sont déplacées sur la peau, le déplacement peut se faire par exemple sans interrompre la circulation du courant entre les électrodes 17 et 18, en maintenant l'excitateur électrique continûment appliqué contre la structure composite 3.

Le cas échéant, l'utilisateur peut faire varier la pression de contact des électrodes sur la structure composite pour faire passer plus ou moins de courant électrique à travers la structure composite dans la peau.

On peut appliquer l'excitateur électrique contre la structure composite 3 immédiatement après avoir posé la structure composite 3 sur la peau, ou après un certain temps, et l'enlever juste avant de retirer la structure composite 3, ou encore laisser cette dernière un certain temps en contact avec la peau après le traitement électrique.

La structure composite 3 peut avoir deux modes d'utilisation, l'un sans circulation de courant électrique, et l'autre avec circulation de courant électrique, et permettre deux types de traitement. L'utilisateur peut par exemple alterner un traitement avec application de courant électrique et un traitement sans. L'utilisateur peut par exemple traiter un jour sans l'excitateur puis le lendemain avec l'excitateur.

Le déplacement de l'excitateur électrique 2 relativement à la structure composite 3 peut également se faire en interrompant périodiquement la circulation du courant électrique, en éloignant l'excitateur électrique 2 de la structure composite 3. L'utilisateur peut par exemple procéder par touches successives. Le cas échéant, l'utilisateur peut manipuler l'interrupteur 14 de façon à provoquer le passage du courant électrique par intermittence.

Après le traitement, la structure composite 3 peut être enlevée et jetée.

Dans une variante de réalisation, l'excitateur électrique 2 est configuré pour transmettre des vibrations à des organes de contact 30 et 31, qui peuvent être des électrodes ou en variante ne pas être conducteurs de l'électricité.

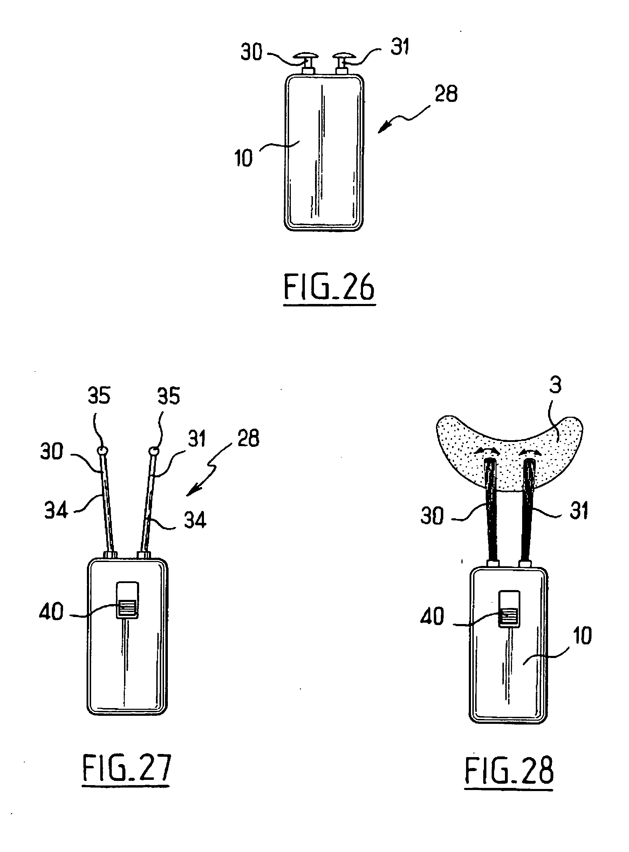

On a illustré à la figure 26 un excitateur électrique 28, comportant des électrodes 30 et 31 qui peuvent être mises en vibration par tout moyen adapté, par exemple un vibreur (non apparent sur les figures) logé dans le boîtier et similaire à ceux rencontrés dans les téléphones portables, comportant un moteur entraînant en rotation une masse non équilibrée. Les vibrations peuvent encore être générées autrement, par exemple par un mécanisme à électroaimant ou piézoélectrique.

Dans l'exemple de la figure 26, les électrodes 30 et 31 ont sensiblement la même forme que les électrodes de l'exemple de réalisation des figures 20 à 25, mais on ne sort pas du cadre de la présente invention s'il en est autrement.

On a représenté sur les figures 27 et 28 un excitateur électrique vibrant 28 dont les électrodes 30 et 31 comportent chacune une tige 34 reliée à l'excitateur, à l'extrémité de laquelle est fixée une tête sensiblement sphérique 35 destinée à entrer en contact avec la structure composite 3.

Les tiges 34 peuvent permettre grâce à leur longueur d'amplifier l'amplitude des vibrations.

Les tiges 34 sont par exemple réalisées dans un acier à ressort, par exemple du genre « corde à piano », dont le diamètre est par exemple compris entre 0,5 et 3 mm, notamment entre 1 et 2 mm.

Les têtes 35 peuvent par exemple être réalisées dans un matériau élastomère conducteur ou être métalliques ou être réalisées dans toute autre matière conductrice de l'électricité.

L'excitateur électrique 28 représenté à la figure 27 à l'arrêt, est représenté à la figure 28 en cours d'utilisation.

La mise en vibration des électrodes 30 et 31 permet d'accroître encore les effets de la structure composite sur la région traitée, par exemple en stimulant localement la circulation sanguine.

Dans l'exemple illustré sur les figures 27 et 28, l'excitateur électrique comporte un sélecteur 40 qui permet à l'utilisateur de choisir entre plusieurs modes de fonctionnement, par exemple un mode avec alimentation électrique des électrodes et absence de mise en vibration, un autre mode avec alimentation électrique des électrodes et mise en vibration et un autre mode encore avec absence d'alimentation électrique des électrodes mais mise en vibration.

Dans une variante non illustrée, l'excitateur électrique comporte un moyen de réglage de l'amplitude et/ou de la fréquence de vibration des électrodes. Il peut s'agir par exemple d'un potentiomètre et de l'électronique associée afin d'alimenter sous une tension et/ou avec une fréquence variable le moteur électrique du vibreur.

Dans toute la description, y compris les revendications, l'expression « comportant un » doit être comprise comme étant synonyme de « comportant au moins un », sauf si le contraire est spécifié.

## Revendications

1. Kit de traitement, comportant :
- une structure composite (3), dépourvue d'électrodes et constituée d'un patch, d'un masque ou d'une serviette, comportant au moins une couche d'un tissé ou d'un non tissé capable d'être rendue conductrice de l'électricité et comportant au moins un actif destiné à exercer une action sur la zone de peau à traiter (P),
- un excitateur électrique (2) comportant au moins deux électrodes (17, 18 ; 30, 31) solidaires d'un organe de préhension (10) permettant de les amener simultanément au contact d'une face extérieure (5) de la structure composite (3) une fois celle-ci appliquée sur la peau afin de faire passer un courant électrique dans celle-ci et de les déplacer par rapport à la structure composite durant le traitement.

2. Kit selon l'une des revendications précédentes, la structure composite (3) comportant :
- une première face (4) à appliquer sur la zone à traiter (P),
- une deuxième face (5) opposée à la première et sur laquelle les électrodes (17, 18 ; 30, 31) peuvent être appliquées,
l'actif étant contenu dans la structure composite entre les première et deuxième faces, et la structure composite (3) étant agencée pour privilégier la migration de l'actif vers la première face (4) plutôt que vers la seconde face (5).

3. Kit selon la revendication précédente, **caractérisé par le fait que** les deux électrodes (17, 18 ; 30, 31) peuvent être amenées simultanément par le maniement de l'organe de préhension (10) au contact de la deuxième face (5) de la structure composite (3).

4. Kit selon l'une quelconque des revendications précédentes, **caractérisé par le fait que** l'excitateur électrique (2) et la structure composite (3) sont configurés de telle sorte que les électrodes (17, 18 ; 30, 31) peuvent venir au contact de la face (5) de la structure composite (3) qui leur est associée sans contact substantiel avec l'actif.

5. Kit selon l'une quelconque des revendications précédentes, **caractérisé par le fait que** la structure composite comporte une couche extérieure (8) hydrophile, apte à être mise en contact avec les électrodes (17, 18 ; 30, 31).

6. Kit selon l'une quelconque des revendications précédentes, **caractérisé par le fait que** la structure composite (3) comporte au moins une couche formée d'un tissé.

7. Kit selon l'une quelconque des revendications précédentes, **caractérisé par le fait que** la structure composite (3) comporte au moins une couche formée d'un non-tissé, notamment deux couches extrêmes (7, 8) formées chacune d'un non-tissé.

8. Kit selon l'une quelconque des revendications précédentes, **caractérisé par le fait que** la structure composite (3) comporte au moins une couche d'une matrice gélifiée et/ou polymérique.

9. Kit selon l'une quelconque des revendications précédentes, **caractérisé par le fait que** la structure composite (3) comporte une couche réservoir (6) contenant l'actif, prise en sandwich entre deux couches (7, 8) dont l'une au moins est dépourvue d'actif avant l'utilisation.

10. Kit selon l'une quelconque des revendications précédentes, **caractérisé par le fait que** la structure composite comporte une couche réservoir contenant l'actif, prise en sandwich entre une couche hydrophile et une couche hydrophile ou hydrophobe.

11. Kit selon l'une quelconque des revendications 1 à 10, **caractérisé par le fait que** la structure composite comporte une couche réservoir (6) comportant l'actif, située entre deux couches (7, 8) d'épaisseurs et/ou de porosités différentes.

12. Kit selon l'une quelconque des revendications 1 à 11, **caractérisé par le fait que** la couche réservoir comporte un adhésif.

13. Kit selon l'une quelconque des revendications 1 à 11, **caractérisé par le fait que** l'une au moins des couches (7, 8) est soudée sur la couche réservoir.

14. Kit selon la revendication précédente, **caractérisé par le fait que** la couche (8) d'épaisseur la plus importante définit la face (5) associée aux électrodes (17, 18 ; 30, 31).

15. Kit selon l'une des deux revendications précédentes, **caractérisé par le fait que** la couche (8) de porosité la plus faible définit la face (5) associée aux électrodes (17, 18;30,31).

16. Kit selon l'une quelconque des revendications précédentes, **caractérisé par le fait que** la structure composite (3) est sensiblement sèche avant l'utilisation.

17. Kit selon l'une quelconque des revendications précédentes, **caractérisé par le fait qu'**avant l'utilisation l'actif est à l'état déshydraté et/ou lyophilisé et/ou cristallisé.

18. Kit selon l'une quelconque des revendications 1 à 13, **caractérisé par le fait que** la structure composite (3) comporte une couche imprégnée d'un liquide, notamment l'un au moins d'eau, d'alcool ou de propylène glycol.

19. Kit selon la revendication précédente, **caractérisé par le fait que** la couche imprégnée de liquide comporte l'actif.

20. Kit selon l'une quelconque des revendications précédentes, **caractérisé par le fait que** la structure composite (3) est non-adhésive à sec et adhésive lorsqu'elle est humidifiée.

21. Kit selon l'une quelconque des revendications précédentes, **caractérisé par le fait que** la structure composite (3) comporte des particules magnétiques.

22. Kit selon l'une quelconque des revendications précédentes, **caractérisé par le fait que** la structure composite (3) comporte des particules électriquement conductrices.

23. Kit selon la revendication 22, **caractérisé par le fait que** la structure composite comporte des particules d'un métal, notamment du cuivre.

24. Kit selon l'une quelconque des revendications 21 à 23, **caractérisé par le fait que** les particules sont localisées dans une couche de la structure composite, notamment dans une couche réservoir (6) contenant l'actif et/ou dans ou entre les fibres d'un tissé ou d'un non-tissé de la structure composite.

25. Kit selon l'une quelconque des revendications précédentes, **caractérisé par le fait que** l'actif comporte au moins un composé choisi dans la liste suivante : métaux et leurs alliages, cobalt, baryum, chrome, aluminium, argent, cuivre, titane, bronze, manganèse, oxydes métalliques, oxydes de fer, notamment ferrite, terres rares, silicates, sulfates, notamment sulfate de baryum, carbonates, notamment carbonate de calcium, composés non ferreux, notamment soufre, magnésium, calcium, bore, potassium, carbone, oligo-éléments, sel marin, sels gemmes, argile, stéatite, algues et planctons et leurs extraits, racines, réglisse, gingembre, cires huileuses, protéines, hormones, collagènes, aluns, notamment pierre d'alun, glucose, vitamines, notamment vitamine C, vitamine A, vitamine F, vitamine B, vitamine E, extraits végétaux, glycérine, laponite, tensioactifs, collagène, acides, notamment acide salicylique, acide tio, caféine, huiles essentielles aromatiques, colorants, anti-oxydants, anti-radicaux libres, hydroabsorbants, hydratants, dépigmentants, liporégulateurs, anti-acnéique, anti-séborrhéiques, agents anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs et nourrissants.

26. Kit selon l'une quelconque des revendications précédentes, **caractérisé par le fait que** la structure composite comporte une couche réservoir contenant l'actif, cette couche réservoir comportant l'un au moins d'un composé superabsorbant, d'un polyacrylate, d'un polymère acrylique, vinylique, d'un polyuréthane, d'un latex, d'un pseudolatex.

27. Kit selon la revendication précédente, **caractérisé par le fait que** la structure composite comporte de la glycérine.

28. Kit selon l'une quelconque des revendications précédentes, **caractérisé par le fait que** les électrodes (17, 18 ; 30, 31) sont montées avec une possibilité de déplacement par rapport au reste de l'excitateur (2), notamment de coulissement et/ou de rotation.

29. Kit selon l'une quelconque des revendications précédentes, **caractérisé par le fait que** l'écartement entre au moins deux électrodes (17, 18) est réglable.

30. Kit selon l'une quelconque des revendications précédentes, **caractérisé par le fait qu'**au moins une électrode est amovible.

31. Kit selon l'une quelconque des revendications 1 à 29, **caractérisé par le fait que** les électrodes (17, 18) sont maintenues immobiles l'une par rapport à l'autre par un isolant électrique (19), notamment un matériau non conducteur choisi dans la liste suivante : résine thermoplastique ou élastomère, notamment silicone.

32. Kit selon l'une quelconque des revendications précédentes, **caractérisé par le fait que** les électrodes (17, 18 ; 30, 31) sont dépourvues d'arête vive.

33. Kit selon l'une quelconque des revendications précédentes, **caractérisé par le fait que** chaque électrode (17, 18 ; 30, 31) comporte une extrémité arrondie, notamment sphérique.

34. Kit selon l'une quelconque des revendications précédentes, **caractérisé par le fait que** l'excitateur électrique (2) présente une forme ergonomique.

35. Kit selon l'une quelconque des revendications 1 à 33, **caractérisé par le fait que** l'excitateur électrique (2) comporte un organe de préhension constitué au moins en partie par une pile (11) ou un accumulateur.

36. Kit selon l'une quelconque des revendications précédentes, **caractérisé par le fait que** l'excitateur électrique comporte un boîtier (10) logeant l'un au moins d'une pile électrique (11) et d'un accumulateur.

37. Kit selon l'une quelconque des revendications 1 à 35, **caractérisé par le fait que** l'excitateur électrique (2) comporte des moyens de raccordement à un adaptateur (21) électrique branché sur le secteur.

38. Kit selon l'une quelconque des revendications précédentes, **caractérisé par le fait que** lors de l'utilisation la tension aux bornes des électrodes (17, 18 ; 30, 31) est identique à la tension aux bornes d'une source électrique (11) logée dans l'excitateur électrique.

39. Kit selon l'une quelconque des revendications précédentes, **caractérisé par le fait que** la tension aux bornes des électrodes (17, 18 ; 30, 31) est inférieure à 30 V, notamment est voisine de 9 V.

40. Kit selon l'une quelconque des revendications précédentes, **caractérisé par le fait que** l'excitateur électrique comporte un organe de réglage de la puissance électrique.

41. Kit selon la revendication 1, **caractérisé par le fait que** la structure composite est un patch au travers duquel le courant électrique peut circuler dans la peau.

42. Kit selon l'une quelconque des revendications précédentes, dans lequel l'excitateur électrique comporte des moyens de mise en vibration des électrodes (30, 31).

43. Kit selon la revendication 42, **caractérisé par le fait que** l'excitateur comporte un sélecteur (40) permettant à l'utilisateur de choisir s'il souhaite ou non faire vibrer les électrodes.

44. Kit selon la revendication 42, **caractérisé par le fait que** l'excitateur comporte un sélecteur (40) permettant le choix d'un mode de fonctionnement dans lequel les électrodes peuvent vibrer sans que celles-ci ne soient alimentées électriquement.

45. Kit selon l'une quelconque des revendications précédentes, l'excitateur électrique comportant une pile ou un accumulateur électrique et une tête (16) pouvant être rendue rigidement solidaire de la pile (11) ou de l'accumulateur, cette tête maintenant lesdites au moins deux électrodes immobiles l'une par rapport à l'autre et permettant de les amener simultanément au contact de la structure composite.

46. Kit selon l'une quelconque des revendications 1 à 44, l'excitateur électrique comportant :
- un boîtier en matière plastique pour recevoir au moins une pile ou accumulateur électrique,
les deux électrodes étant fixées sur le boîtier et comportant chacune une tête extérieure arrondie et une tige (34 ; 41) venant en contact mécanique avec une borne de la pile ou de l'accumulateur.

47. Procédé de traitement cosmétique, non thérapeutique, d'une zone de la peau, comportant les étapes suivantes :
- appliquer sur la zone de la peau à traiter une structure composite (3)
dépourvue d'électrodes et constituée d'un patch, d'un masque ou d'une serviette, comportant au moins une couche d'un tissé ou d'un non tissé capable d'être rendue conductrice de l'électricité et comportant au moins un actif destiné à exercer une action sur la zone de peau à traiter,
- amener au contact de la structure composite (3) un excitateur électrique (2) comportant au moins deux électrodes (17, 18 ; 30, 31), la structure composite (3) étant agencée pour permettre le passage d'un courant dans la structure composite sans contact substantiel des électrodes (17, 18) avec l'actif,
les électrodes étant déplacées par rapport à la structure composite (3) durant le traitement.

48. Procédé selon la revendication précédente, **caractérisé par le fait qu'**il comporte en outre l'étape suivante :
- humidifier la structure composite (3) avant son application sur la zone à traiter.

49. Procédé selon la revendication 47 ou 48, **caractérisé par le fait que** la structure composite devient adhésive sur la peau.

50. Procédé selon l'une des deux revendications précédentes, **caractérisé par le fait qu'**il comporte en outre l'étape suivante :
- humidifier la peau préalablement à l'application de la structure composite (3).

51. Procédé selon la revendication 50, **caractérisé par le fait qu'**on applique la structure composite (3) sèche sur une zone de la peau préalablement humidifiée.

52. Procédé selon l'une quelconque des revendications 47 à 51, **caractérisé par le fait qu'**on applique les électrodes sur la structure composite (3) de façon permanente durant tout le traitement.

53. Procédé selon l'une quelconque des revendications 47 à 51, **caractérisé par le fait qu'**on applique les électrodes (17, 18) sur la structure composite (3) de façon intermittente durant le traitement.

54. Procédé selon l'une quelconque des revendications 47 à 53, **caractérisé par le fait qu'**on applique au total les électrodes sur la structure composite (3) pendant une durée comprise entre 3 et 15 mn.

55. Procédé selon l'une quelconque des revendications 47 à 54, **caractérisé par le fait que** la structure composite (3) est jetée après utilisation.

56. Procédé selon l'une quelconque des revendications 47 à 55, **caractérisé par le fait que** l'on met en vibration les électrodes durant le traitement.

## Claims

1. A treatment kit comprising:
· a composite structure (3) deprived of any electrodes and being one of a patch, a mask, and a cloth, comprising at least one layer formed by a woven fabric or a non-woven fabric, suitable for being made electrically conductive, and comprising at least one active agent for exerting an action on a zone (P) of the skin to be treated; and
· an electric exciter (2) comprising at least two electrodes (17, 18; 30, 31) secured to a handle (10) enabling them to be brought simultaneously into contact with an outside surface (5) of the composite structure (3) once the composite structure is applied on the skin, so as to cause an electric current to pass into said composite structure and to displace them with regard to the composite structure during treatment.

2. A kit according to one of the preceding claim the composite structure comprising:
· a first surface (4) for applying to the zone (P) to be treated;
· a second surface (5) opposite from the first and against which the electrodes (17, 18; 30, 31) can be applied;
· said active agent being contained in the composite structure between the first and second surfaces, and the composite structure (3) being arranged to encourage migration of the active agent towards the first surface (4) rather than towards the second surface (5).

3. A kit according to the preceding claim, **characterized by** the fact that both electrodes (17, 18; 30, 31) can be brought simultaneously into contact with the second surface (5) of the composite structure (3) by manipulating the handle (10).

4. A kit according to any preceding claim, **characterized by** the fact that the electric exciter (2) and the composite structure (3) are configured so that the electrodes (17, 18; 30, 31) can come into contact with the surface (5) of the associated composite structure (3), while making no significant contact with the active agent.

5. A kit according to any preceding claim, **characterized by** the fact that the composite structure comprises a hydrophilic outer layer (8) suitable for being put into contact with the electrodes (17, 18; 30, 31).

6. A kit according to any preceding claim, **characterized by** the fact that the composite structure (3) comprises at least one layer formed by a woven fabric.

7. A kit according to any preceding claim, **characterized by** the fact that the composite structure (3) comprises at least one layer formed by a non-woven fabric, in particular two outermost layers (7, 8), which are both formed by a non-woven fabric.

8. A kit according to any preceding claim, **characterized by** the fact that the composite structure (3) comprises at least one layer of a gelled and/or polymeric matrix.

9. A kit according to any preceding claim, **characterized by** the fact that the composite structure (3) comprises a reservoir layer (6) containing the active agent, said layer being sandwiched between two layers (7, 8) at least one of which is free from any active agent before use.

10. A kit according to any preceding claim, **characterized by** the fact that the composite structure comprises a reservoir layer containing the active agent, said layer being sandwiched between a hydrophilic layer and a layer that is hydrophilic or hydrophobic.

11. A kit according to any one of claims 1 to 10, **characterized by** the fact that the composite structure comprises a reservoir layer (6) containing the active agent, said layer being situated between two layers (7, 8) of differing thicknesses and/or porosities.

12. A kit according to any one of claims 1 to 11, **characterized by** the fact that the reservoir layer contains an adhesive.

13. A kit according to any one of claims 1 to 11, **characterized by** the fact that at least one of the layers (7, 8) is bonded onto the reservoir layer.

14. A kit according to the preceding claim, **characterized by** the fact that the thicker layer (8) defines the surface (5) associated with the electrodes (17, 18; 30, 31).

15. A kit according to any one of the two preceding claims, **characterized by** the fact that the layer (8) of lower porosity defines the surface (5) associated with the electrodes (17, 18; 30, 31).

16. A kit according to any preceding claim, **characterized by** the fact that the composite structure (3) is substantially dry before use.

17. A kit according to any preceding claim, **characterized by** the fact that, before use, the active agent is in the dehydrated and/or freeze-dried and/or crystallized state.

18. A kit according to any one of claims 1 to 13, **characterized by** the fact that the composite structure (3) comprises a layer impregnated with a liquid, in particular one of water, alcohol, or propylene glycol.

19. A kit according to the preceding claim, **characterized by** the fact that the liquid-impregnated layer contains the active agent.

20. A kit according to any preceding claim, **characterized by** the fact that the composite structure (3) is non-adhesive when dry and adhesive when dampened.

21. A kit according to any preceding claim, **characterized by** the fact that the composite structure (3) comprises magnetic particles.

22. A kit according to any preceding claim, **characterized by** the fact that the composite structure (3) comprises electrically-conductive particles.

23. A kit according to claim 22, **characterized by** the fact that the composite structure comprises particles of a metal, in particular of copper.

24. A kit according to any one of claims 21 to 23, **characterized by** the fact that the particles are located in a layer of the composite structure, in particular in a reservoir layer (6) containing the active agent, and/or in or between the fibers of a woven or a non-woven fabric of the composite structure.

25. A kit according to any preceding claim, **characterized by** the fact that the active agent comprises at least one compound selected from the following list: metals and their alloys; cobalt; barium; chromium; aluminum; silver; copper; titanium; bronze; manganese; metallic oxides; iron oxides, in particular ferrite; rare earths; silicates; sulfates, in particular barium sulfate; carbonates, in particular calcium carbonates; non-ferrous compounds, in particular of sulfur, magnesium, calcium, boron, potassium, or carbon; oligo-elements; sea salt; rock salt; clay; steatite; algae and plankton and extracts therefrom; roots; liquorice; ginger; oily waxes; proteins; hormones; collagens; alums in particular alum stone; glucose; vitamins, in particular vitamin C, vitamin A, Vitamin F, vitamin B, vitamin E; plant extracts; glycerin; laponite; wetting agents; collagen; acids, in particular salicylic acid; thio acid; caffeine; essential aromatic oils; coloring agents; anti-oxidants; free radical scavengers; moisture absorbers; moisturizers; depigmenting agents; liporegulators; anti-acne agents; antidandruff agents; anti-ageing agents; softeners; antiwrinkle agents; keratolitic agents; antiinflammatory agents; fresheners; healing agents; vascular protectors; antibacterial agents; antifungal agents; antiperspirants; deodorants; skin conditioners; anesthetics; immunomodulators; and nourishing agents.

26. A kit according to any preceding claim, **characterized by** the fact that the composite structure comprises a reservoir layer containing the active agent, said reservoir layer comprising at least one of the following: a super-absorbent compound; a polyacrylate; an acrylic polymer; a vinyl polymer; a polyurethane; a latex; and a pseudolatex.

27. A kit according to the preceding claim, **characterized by** the fact that the composite structure comprises glycerin.

28. A kit according to any preceding claim, **characterized by** the fact that the electrodes (17, 18; 30, 31) are mounted with a possibility of being displaced relative to the rest of the exciter (2), in particular sliding and/or of turning.

29. A kit according to any preceding claim, **characterized by** the fact that the distance between at least two electrodes (17, 18) is adjustable.

30. A kit according to any preceding claim, **characterized by** the fact that at least one electrode is removable.

31. A kit according to any one of claims 1 to 29, **characterized by** the fact that the electrodes (17, 18) are prevented from moving relative to each other by an electrical insulator (19), in particular a non-conductive material selected from the following list: an elastomer, a thermoplastic resin, silicone.

32. A kit according to any preceding claim, **characterized by** the fact that the electrodes (17, 18; 30, 31) have no sharp edges.

33. A kit according to any preceding claim, **characterized by** the fact that each electrode (17, 18; 30, 31) has an end that is rounded, in particular spheric.

34. A kit according to any preceding claim, **characterized by** the fact that the electric exciter (2) presents a shape that is ergonomic.

35. A kit according to any one of claims 1 to 33, **characterized by** the fact that the electric exciter (2) comprises a handle constituted, at least in part, by an optionally rechargeable battery (11).

36. A kit according to any preceding claim, **characterized by** the fact that the electric exciter comprises a box (10) housing at least one optionally rechargeable battery 11).

37. A kit according to any one of claims 1 to 35, **characterized by** the fact that the electric exciter (2) comprises connection means for connecting to an electrical adaptor (21) connected to the mains.

38. A kit according to any preceding claim, **characterized by** the fact that, in use, the voltage at the terminals of the electrodes (17, 18; 30, 31) is identical to the voltage at the terminals of an electricity source (11) housed in the electric exciter.

39. A kit according to any preceding claim, **characterized by** the fact that the voltage at the terminals of the electrodes (17, 18; 30, 31) is less than 30 V, in particular of the order of 9 V.

40. A kit according to any preceding claim, **characterized by** the fact that the electric exciter comprises a control member for adjusting the electrical power.

41. A kit according to claim 1, **characterized by** the fact that the composite structure is a patch through which the electric current can flow into the skin.

42. A kit according to any preceding claim, in which the electric exciter comprises vibrator means for causing the electrodes (30, 31) to vibrate.

43. A kit according to claim 42, **characterized by** the fact that the exciter comprises a selector (40) making it possible for the user to choose whether or not the electrodes are caused to vibrate.

44. A kit according to claim 42, **characterized by** the fact that the exciter comprises a selector (40) making it possible to choose an operating mode in which the electrodes can vibrate, without said electrodes being electrically powered.

45. An electric exciter comprising an optionally rechargeable battery and a head (16) that can be rigidly secured to the optionally rechargeable battery (11), the head holding at least two electrodes that are prevented from moving relative to each other, and said head enabling them to be brought simultaneously into contact with a composite structure.

46. A kit according to any one of claims 1 to 44, the electric exciter comprising:
· a box made of plastics material for receiving at least one optionally rechargeable battery;
the two electrodes being fixed on the box and each comprising an external rounded head and a rod (34; 41) coming into mechanical contact with a terminal of the battery.

47. A non-therapeutical cosmetic-treatment method for cosmetically treating a zone of the skin, said method comprising the following steps:
· applying a composite structure (3) to the zone of the skin to be treated, the structure having no electrodes and being one of a patch, a mask, and a cloth, comprising at least one layer formed by a woven fabric or a non-woven fabric, suitable for being made electrically conductive and comprising et least one active agent for executing an action on a zone of the skin to be treated,
· bringing an electric exciter (2) including at least two electrodes (17, 18; 30, 31) into contact with the composite structure (3), the composite structure (3) being arranged to enable a current to pass into the composite structure with no significant contact between the electrodes (17, 18) and the active agent, the electrodes being displaced with regard to the composite structure during treatment.

48. A method according to the preceding claim, **characterized by** the fact that it further comprises the following step:
· dampening the composite structure (3) before it is applied to the zone to be treated.

49. A method according to claim 47 or claim 48, **characterized by** the fact that the composite structure becomes adhesive on the skin.

50. A method according to any one of the two preceding claims, **characterized by** the fact that it further comprises the following step:
· dampening the skin prior to applying the composite structure (3).

51. A method according to claim 50, **characterized by** the fact that the dry composite structure (3) is applied to a zone of the skin that has been dampened beforehand.

52. A method according to any one of claims 47 to 51, **characterized by** the fact that, the electrodes are applied to the composite structure (3) continuously throughout treatment.

53. A method according to any one of claims 47 to 51, **characterized by** the fact that the electrodes (17, 18) are applied to the composite structure (3) intermittently during treatment.

54. A method according to any one of claims 47 to 53, **characterized by** the fact that the electrodes are applied to the composite structure (3) for a total period of time lying in the range 3 min to 15 min.

55. A method according to any one of claims 47 to 54, **characterized by** the fact that the composite structure (3) is discarded after use.

56. A method according to any one of claims 47 to 55, **characterized by** the fact that, during treatment, the electrodes are caused to vibrate.

## Patentansprüche

1. Behandlungsset, umfassend:
- eine Verbundstruktur (3), die ohne Elektroden ist und aus einem Patch, einer Maske oder einem Tuch besteht, umfassend mindestens eine Schicht eines Gewebes oder Vlieses, das in der Lage ist, elektrisch leitend gemacht zu werden, und umfassend mindestens einen Wirkstoff, der dazu bestimmt ist, auf die zu behandelnde Hautzone (P) eine Wirkung auszuüben,
- einen elektrischen Erreger (2), umfassend mindestens zwei Elektroden (17, 18; 30, 31), die mit einem Greiforgan (10) fest verbunden sind, das es gestattet, sie gleichzeitig mit einer äußeren Seite (5) der Verbundstruktur (3) in Kontakt zu bringen, wenn diese auf die Haut aufgelegt ist, um in dieser einen elektrischen Strom fließen zu lassen, und sie während der Behandlung bezüglich der Verbundstruktur zu bewegen.

2. Set nach einem der vorhergehenden Ansprüche, wobei die Verbundstruktur (3) umfasst:
- eine erste Seite (4), die auf die zu behandelnde Zone (P) aufzulegen ist,
- eine zweite, der ersten entgegengesetzte Seite (5), auf die die Elektroden (17, 18; 30, 31) aufgelegt werden können,
wobei der Wirkstoff in der Verbundstruktur zwischen der ersten Seite und der zweiten Seite enthalten ist und die Verbundstruktur (3) ausgebildet ist, um dem Wandern des Wirkstoffs auf die erste Seite (4) zu gegenüber dem Wandern auf die zweite Seite (5) zu den Vorrang zu geben.

3. Set nach dem vorhergehenden Anspruch, **dadurch gekennzeichnet, dass** die beiden Elektroden (17, 18; 30, 31) gleichzeitig durch die Handhabung des Greiforgans (10) mit der zweiten Seite (5) der Verbundstruktur (3) in Kontakt gebracht werden können.

4. Set nach einem der vorhergehenden Ansprüche, **dadurch gekennzeichnet, dass** der elektrische Erreger (2) und die Verbundstruktur (3) so ausgebildet sind, dass die Elektroden (17, 18; 30, 31) mit der Seite (5) der Verbundstruktur (3), die ihnen zugeordnet ist, ohne substantiellen Kontakt mit dem Wirkstoff in Kontakt kommen können.

5. Set nach einem der vorhergehenden Ansprüche, **dadurch gekennzeichnet, dass** die Verbundstruktur eine hydrophile äußere Schicht (8) umfasst, die in der Lage ist, mit den Elektroden (17, 18; 30, 31) in Kontakt gebracht zu werden.

6. Set nach einem der vorhergehenden Ansprüche, **dadurch gekennzeichnet, dass** die Verbundstruktur (3) mindestens eine von einem Gewebe gebildete Schicht umfasst.

7. Set nach einem der vorhergehenden Ansprüche, **dadurch gekennzeichnet, dass** die Verbundstruktur (3) mindestens eine von einem Vlies gebildete Schicht umfasst, und zwar insbesondere zwei Außenschichten (7, 8), die jeweils von einem Vlies gebildet sind.

8. Set nach einem der vorhergehenden Ansprüche, **dadurch gekennzeichnet, dass** die Verbundstruktur (3) mindestens eine Schicht aus einer gelierten und/oder polymerischen Matrix umfasst.

9. Set nach einem der vorhergehenden Ansprüche, **dadurch gekennzeichnet, dass** die Verbundstruktur (3) eine den Wirkstoff enthaltende Speicherschicht (6) umfasst, die sandwichartig zwischen zwei Schichten (7, 8) ergriffen ist, von denen mindestens eine vor der Verwendung frei von Wirkstoff ist.

10. Set nach einem der vorhergehenden Ansprüche, **dadurch gekennzeichnet, dass** die Verbundstruktur eine den Wirkstoff enthaltende Speicherschicht umfasst, die sandwichartig zwischen einer hydrophilen Schicht und einer hydrophilen oder hydrophoben Schicht ergriffen ist.

11. Set nach einem der Ansprüche 1 bis 10, **dadurch gekennzeichnet, dass** die Verbundstruktur eine den Wirkstoff umfassende Speicherschicht (6) umfasst, die zwischen zwei Schichten (7, 8) von verschiedenen Dicken und/oder Porositäten gelegen ist.

12. Set nach einem der Ansprüche 1 bis 11, **dadurch gekennzeichnet, dass** die Speicherschicht einen Klebstoff umfasst.

13. Set nach einem der Ansprüche 1 bis 11, **dadurch gekennzeichnet, dass** mindestens eine der Schichten (7, 8) an die Speicherschicht angeschweißt ist.

14. Set nach dem vorhergehenden Anspruch, **dadurch gekennzeichnet, dass** die Schicht (8) mit der größten Dicke die den Elektroden (17, 18; 30, 31) zugeordnete Seite (5) bildet.

15. Set nach einem der beiden vorhergehenden Ansprüche, **dadurch gekennzeichnet, dass** die Schicht (8) mit der geringsten Porosität die den Elektroden (17, 18; 30, 31) zugeordnete Seite (5) bildet.

16. Set nach einem der vorhergehenden Ansprüche, **dadurch gekennzeichnet, dass** die Verbundstruktur (3) vor der Verwendung im Wesentlichen trocken ist.

17. Set nach einem der vorhergehenden Ansprüche, **dadurch gekennzeichnet, dass** der Wirkstoff vor der Behandlung im dehydratisierten und/oder lyophilisierten und/oder kristallisierten Zustand ist.

18. Set nach einem der Ansprüche 1 bis 13, **dadurch gekennzeichnet, dass** die Verbundstruktur (3) eine Schicht umfasst, die mit einer Flüssigkeit getränkt ist, insbesondere mit mindestens einem von Wasser, Alkohol oder Propylenglycol.

19. Set nach dem vorhergehenden Anspruch, **dadurch gekennzeichnet, dass** die mit Flüssigkeit getränkte Schicht den Wirkstoff umfasst.

20. Set nach einem der vorhergehenden Ansprüche, **dadurch gekennzeichnet, dass** die Verbundstruktur (3) trocken nicht klebend ist und klebend ist, wenn sie befeuchtet ist.

21. Set nach einem der vorhergehenden Ansprüche, **dadurch gekennzeichnet, dass** die Verbundstruktur (3) magnetische Teilchen umfasst.

22. Set nach einem der vorhergehenden Ansprüche, **dadurch gekennzeichnet, dass** die Verbundstruktur (3) elektrisch leitende Teilchen umfasst.

23. Set nach Anspruch 22, **dadurch gekennzeichnet, dass** die Verbundstruktur Teilchen aus einem Metall, insbesondere Kupfer, umfasst.

24. Set nach einem der Ansprüche 21 bis 23, **dadurch gekennzeichnet, dass** die Teilchen in einer Schicht der Verbundstruktur lokalisiert sind, insbesondere in einer den Wirkstoff enthaltenden Speicherschicht (6) und/oder in oder zwischen den Fasern eines Gewebes oder Vlieses der Verbundstruktur.

25. Set nach einem der vorhergehenden Ansprüche, **dadurch gekennzeichnet, dass** der Wirkstoff mindestens eine Verbindung umfasst, die aus der folgenden Liste ausgewählt ist: Metalle und ihre Legierungen, Kobalt, Barium, Chrom, Aluminium, Silber, Kupfer, Titan, Bronze, Mangan, Metalloxide, Eisenoxide, insbesondere Ferrit, Seltene Erden, Silicate, Sulfate, insbesondere Bariumsulfat, Carbonate, insbesondere Calciumcarbonat, Nichteisenverbindungen, insbesondere Schwefel, Magnesium, Calcium, Bor, Kalium, Kohlenstoff, Oligoelemente, Meersalz, Steinsalze, Ton, Steatit, Algen und Plankton und ihre Extrakte, Wurzeln, Süßholz, Ingwer, Ölwachse, Proteine, Hormone, Collagene, Alaune, insbesondere Alaunstein, Glucose, Vitamine, insbesondere Vitamin C, Vitamin A, Vitamin F, Vitamin B, Vitamin E, Pflanzenextrakte, Glycerin, Laponit, Tenside, Collagen, Säuren, insbesondere Salicylsäure, Thiosäure, Coffein, aromatische essentielle Öle, Farbstoffe, Antioxidantien, Mittel gegen freie Radikale, Wasser absorbierende Mittel, hydratisierende Mittel, Depigmentierungsmittel, Fett regulierende Mittel, Antiaknemittel, Anti-Seborrhöe-Mittel, Mittel gegen Altern, reizlindernde Mittel, Mittel gegen Falten, keratolytische Mittel, entzündungshemmende Mittel, erfrischende Mittel, Vernarbungsmittel, Gefäße schützende Mittel, antibakterielle Mittel, antifungische Mittel, Antiperspirants, Deodorants, Hautkonditionierungsmittel, Desensibilisierungsmittel, Immunomodulatoren und Nährstoffe.

26. Set nach einem der vorhergehenden Ansprüche, **dadurch gekennzeichnet, dass** die Verbundstruktur eine den Wirkstoff enthaltende Speicherschicht umfasst, wobei diese Speicherschicht mindestens eines einer superabsorbierenden Verbindung, eines Polyacrylats, eines acrylischen, vinylischen Polymers, eines Polyurethans, eines Latex, eines Pseudolatex umfasst.

27. Set nach dem vorhergehenden Anspruch, **dadurch gekennzeichnet, dass** die Verbundstruktur Glycerin umfasst.

28. Set nach einem der vorhergehenden Ansprüche, **dadurch gekennzeichnet, dass** die Elektroden (17, 18; 30, 31) mit einer Möglichkeit der Bewegung bezüglich des Rests des Erregers (2), insbesondere des Gleitens und/oder der Drehung, montiert sind.

29. Set nach einem der vorhergehenden Ansprüche, **dadurch gekennzeichnet, dass** der Abstand zwischen mindestens zwei Elektroden (17, 18) verstellbar ist.

30. Set nach einem der vorhergehenden Ansprüche, **dadurch gekennzeichnet, dass** mindestens eine Elektrode abnehmbar ist.

31. Set nach einem der Ansprüche 1 bis 29, **dadurch gekennzeichnet, dass** die Elektroden (17, 18) in Bezug aufeinander unbeweglich gehalten werden durch einen elektrischen Isolierstoff (19), insbesondere einen nicht leitenden Werkstoff, der aus der folgenden Liste ausgewählt ist: thermoplastisches Harz oder Elastomer, insbesondere Silicon.

32. Set nach einem der vorhergehenden Ansprüche, **dadurch gekennzeichnet, dass** die Elektroden (17, 18; 30, 31) ohne scharfe Kante sind.

33. Set nach einem der vorhergehenden Ansprüche, **dadurch gekennzeichnet, dass** jede Elektrode (17, 18; 30, 31) ein abgerundetes, insbesondere kugelförmiges Ende umfasst.

34. Set nach einem der vorhergehenden Ansprüche, **dadurch gekennzeichnet, dass** der elektrische Erreger (2) eine ergonomische Form aufweist.

35. Set nach einem der Ansprüche 1 bis 33, **dadurch gekennzeichnet, dass** der elektrische Erreger (2) ein Greiforgan umfasst, das mindestens zum Teil aus einer Batterie (11) oder einem Akkumulator besteht.

36. Set nach einem der vorhergehenden Ansprüche, **dadurch gekennzeichnet, dass** der elektrische Erreger ein Gehäuse (10) umfasst, das mindestens eines einer elektrischen Batterie (11) und eines Akkumulators beherbergt.

37. Set nach einem der Ansprüche 1 bis 35, **dadurch gekennzeichnet, dass** der elektrische Erreger (2) Mittel zur Verbindung mit einem an das Netz angeschlossenen elektrischen Adapter (21) umfasst.

38. Set nach einem der vorhergehenden Ansprüche, **dadurch gekennzeichnet, dass** die Spannung an den Anschlüssen der Elektroden (17, 18; 30, 31) bei der Verwendung identisch mit der Spannung an den Anschlüssen einer in dem elektrischen Erreger untergebrachten Stromquelle (11) ist.

39. Set nach einem der vorhergehenden Ansprüche, **dadurch gekennzeichnet, dass** die Spannung an den Anschlüssen der Elektroden (17, 18; 30, 31) niedriger als 30 V, insbesondere nahe 9 V ist.

40. Set nach einem der vorhergehenden Ansprüche, **dadurch gekennzeichnet, dass** der elektrische Erreger ein Organ zum Einstellen der elektrischen Leistung umfasst.

41. Set nach Anspruch 1, **dadurch gekennzeichnet, dass** die Verbundstruktur ein Patch ist, durch den der elektrische Strom in die Haut fließen kann.

42. Set nach einem der vorhergehenden Ansprüche, bei dem der elektrische Erreger Mittel umfasst, um die Elektroden (30, 31) in Vibration zu versetzen.

43. Set nach Anspruch 42, **dadurch gekennzeichnet, dass** der Erreger einen Wähler (40) umfasst, der dem Benutzer gestattet, die Elektroden, wenn er es wünscht oder nicht in Vibration zu versetzen.

44. Set nach Anspruch 42, **dadurch gekennzeichnet, dass** der Erreger einen Wähler (40) umfasst, der die Wahl einer Betriebsart gestattet, bei der die Elektroden vibrieren können, ohne dass sie elektrisch versorgt werden.

45. Set nach einem der vorhergehenden Ansprüche, wobei der elektrische Erreger eine Batterie oder einen elektrischen Akkumulator und einen Kopf (16) umfasst, der mit der Batterie (11) oder dem Akkumulator starr fest verbunden werden kann, wobei dieser Kopf die mindestens zwei Elektroden in Bezug aufeinander unbeweglich hält und es gestattet, sie gleichzeitig mit der Verbundstruktur in Kontakt zu bringen.

46. Set nach einem der Ansprüche 1 bis 44, wobei der elektrische Erreger umfasst:
- ein Gehäuse aus Kunststoff, um mindestens eine Batterie oder einen elektrischen Akkumulator aufzunehmen,
wobei die beiden Elektroden an dem Gehäuse befestigt sind und jeweils einen abgerundeten äußeren Kopf und eine Stange (34; 41) umfassen, die mit einem Pol der Batterie oder des Akkumulators in mechanischen Kontakt kommt.

47. Verfahren zum nicht therapeutischen kosmetischen Behandeln einer Zone der Haut, umfassend die folgenden Schritte:
- auf die zu behandelnde Zone der Haut eine von Elektroden freie Verbundstruktur (3) aufbringen, die aus einem Patch, einer Maske oder einem Tuch besteht, umfassend mindestens eine Schicht eines Gewebes oder eines Vlieses, das in der Lage ist, elektrisch leitend gemacht zu werden, und umfassend mindestens einen Wirkstoff, der dazu bestimmt ist, auf die zu behandelnde Hautzone eine Wirkung auszuüben,
- mit der Verbundstruktur (3) einen elektrischen Erreger (2) in Kontakt bringen, der mindestens zwei Elektroden (17, 18; 30, 31) umfasst, wobei die Verbundstruktur (3) ausgebildet ist, um den Durchgang eines Stroms in der Verbundstruktur ohne substantiellen Kontakt der Elektroden (17, 18) mit dem Wirkstoff zu gestatten,
wobei die Elektroden während der Behandlung bezüglich der Verbundstruktur (3) bewegt werden.

48. Verfahren nach dem vorhergehenden Anspruch, **dadurch gekennzeichnet, dass** es außerdem den folgenden Schritt umfasst:
- die Verbundstruktur (3) vor ihrem Auflegen auf die zu behandelnde Zone befeuchten.

49. Verfahren nach Anspruch 47 oder 48, **dadurch gekennzeichnet, dass** die Verbundstruktur auf der Haut klebend wird.

50. Verfahren nach einem der beiden vorhergehenden Ansprüche, **dadurch gekennzeichnet, dass** es außerdem den folgenden Schritt umfasst:
- die Haut vor dem Auflegen der Verbundstruktur (3) befeuchten.

51. Verfahren nach Anspruch 50, **dadurch gekennzeichnet, dass** man die trockene Verbundstruktur (3) auf eine zuvor befeuchtete Zone der Haut auflegt.

52. Verfahren nach einem der Ansprüche 47 bis 51, **dadurch gekennzeichnet, dass** man die Elektroden auf die Verbundstruktur (3) während der gesamten Behandlung permanent auflegt.

53. Verfahren nach einem der Ansprüche 47 bis 51, **dadurch gekennzeichnet, dass** man die Elektroden (17, 18) auf die Verbundstruktur (3) während der Behandlung intermittierend auflegt.

54. Verfahren nach einem der Ansprüche 47 bis 53, **dadurch gekennzeichnet, dass** man die Elektroden an die Verbundstruktur (3) insgesamt während einer Dauer zwischen 3 und 15 min anlegt.

55. Verfahren nach einem der Ansprüche 47 bis 54, **dadurch gekennzeichnet, dass** die Verbundstruktur (3) nach Verwendung weggeworfen wird.

56. Verfahren nach einem der Ansprüche 47 bis 55, **dadurch gekennzeichnet, dass** man die Elektroden während der Behandlung in Vibration versetzt.
